# EUROPÄISCHE PATENTSCHRIFT

(11) **EP 1 084 716 B1**
(45) Veröffentlichungstag und Bekanntmachung des Hinweises auf die Patenterteilung: **04.01.2006**
(21) Anmeldenummer: 00110783.8
(22) Anmeldetag: 20.05.2000
(51) Int. Cl.: A61L 15/44

(54) **Wundauflage zur gesteuerten Abgabe von Wirkstoff an Wunden und Verfahren zu ihrer Herstellung**
Dressing for the controlled release of active substances to wounds and method for its manufacture
Pansement pour blessure à libération contrôlée de principes actifs et son procédé de fabrication

(30) Priorität: 04.06.1999 DE 19925519
(43) Veröffentlichungstag der Anmeldung: 21.03.2001
(73) Patentinhaber: LTS LOHMANN Therapie-Systeme AG, 56626 Andernach (DE)
(72) Erfinder: Berthold, Achim, Dr., 56626 Andernach (DE); Müller, Walter, Dr., 56564 Neuwied (DE); Flöther, Ulrich, 8207 Schaffhausen (DE); Naeff, Rainer, Dr., 8246 Langwiesen (CH)
(74) Vertreter: Flaccus, Rolf-Dieter

(56) Entgegenhaltungen:
- WO-A-98/28013
- US-A- 4 608 249
- US-A- 5 578 661
- US-A- 5 705 485
- DATABASE WPI Section Ch, Week 198404 Derwent Publications Ltd., London, GB; Class A96, AN 1984-021526 XP002189850 & JP 58 213709 A (TEIKOKU SEIYAKU KK), 12. Dezember 1983 (1983-12-12)
- DATABASE WPI Section Ch, Week 198540 Derwent Publications Ltd., London, GB; Class A96, AN 1985-247023 XP002189851 & JP 60 163811 A (WATANABE YAKUHIN KOGYO KK), 26. August 1985 (1985-08-26)

## Beschreibung

Die Erfindung betrifft eine Wundauflage zur gesteuerten Abgabe von Wirkstoff an Wunden, insbesondere von wundheilungsfördernden Substanzen an schlecht heilende chronische Wunden sowie ein Verfahren zu ihrer Herstellung.

Definitionsgemäß ist jede Wunde, die innerhalb von acht Wochen keine Tendenz zur Heilung zeigt, als chronisch anzusehen.
Hinsichtlich aktueller Wundtherapie hat eine Wundauflage die folgenden Therapieaspekte zu berücksichtigen:
- Sie soll ein feucht-warmes Wundmilieu unter weitgehendem Ausschluß von atmosphärischem Sauerstoff schaffen, weil Wunden unter Okklusion schneller heilen. Als Ursache für die "heilende" Wirkung eines okklusiven Milieus wird in der Literatur unter anderen folgender Mechanismus diskutiert: Durch Sauerstoffausschluß ist die Wunde gezwungen, über das Blut Sauerstoff in das Wundgebiet zu bringen. Dies erfolgt durch vermehrte Gefäßeinsprossung unter Gefäßneubildung und damit geförderte Wundheilung.
- Um den Heilungsprozeß nicht zu stören, sollte die Wundauflage nur selten gewechselt werden, weil das Abnehmen einer vollgesaugten Wundauflage einen Verlust von Bestandteilen des Wundexsudats und damit immunkompetenter Zellen verursacht.
- Alles, womit die körpereigenen Abwehr- und Reinigungsmechanismen nicht fertig werden, muß aus der Wunde entfernt werden. Besonders nicht abfließendes, eventuell mit Keimen besetztes Sekret birgt ein hohes Infektionsrisiko. Es bildet sich eine feuchte Kammer. Womöglich wird dieser negative Effekt noch durch einen ungeeigneten Verband mit schlechter Saugkraft und Wasserdampfdurchlässigkeit verstärkt.

Wundauflagen lassen sich aufgrund ihrer Eigenschaften in verschiedene Gruppen einteilen. Es werden unterschieden:
- Interaktive Wundauflagen: Hydrokolloide, Hydrogele, Alginate, Polyurethan-Schäume und Folien.
- Aktive Wandauflagen: Aktivkohle-Kompressen, Silber-Aktivkohle-Kompressen und Jodgaze. Sie greifen durch absorptive Vorgänge aktiv ins Wundklima ein und haben einen chemisch-physiklisch definierten Wirkungsmechanismus, aber keine nennenswerte Saugleistung für Sekret.
- Klassifiziert man auch nach Atmungsfähigkeit, so gehören die Alginate, Hydrokolloid-Gele, Hydrogele und Spezialfolien zu den gaspermeablen Systemen und die Hydrokolloid-, Hydrogel- und Weichschaum-Kompressen sowie die Standardfolien zu den somipermeablen/semiokklusiven Systemen.

Untersuchungen haben ergeben, daß im Vergleich zu akuten Wunden in chronischen Wunden erheblich mehr Leukozyten und Matrixmetalloproteinasen (MMP) zu finden sind. Durch erhöhte Konzentration an MMP wird die extrazelluläre Matrix abgebaut und zudem Wachstumsfaktoren, einschließlich ihrer Rezepturen, an den Zielzellen degradiert, so daß die Wundheilungskaskade ins Stocken gerät. Durch die externe Zufuhr von Wundheilungsfaktoren, insbesondere von Wachstumsfaktoren, kann der Teufelskreis durchbrochen werden und die Wundheilungskaskade wieder anlaufen.

Der Einsatz von Gelen, insbesondere von Hydrogelen, zur Behandlung von geschädigter Haut und Wunden wird in der Literatur beschrieben. Vorteilhaft bei ihrem Einsatz ist, daß sie eine gute biologische Verträglichkeit aufweisen, insbesondere dann, wenn sie über einen längeren Zeitraum appliziert werden. Hierbei werden für die Herstellung unterschiedliche Substanzen, Hilfsstoffe und Wirkstoffe eingesetzt.

Gele können allein oder zusammen mit Wirkstoffen eingesetzt werden. Werden Gele als solche z.B. ohne Wirkstoff eingesetzt, dann dienen sie vor allem der Regulierung des Wundmilieus. Ziel ist es, ein Wundmilieu zu schaffen, das die Wundheilung fördert. Dies beruht auf der Erkenntnis, daß Wunden unter feucht-warmen Bedingungen besser und schneller heilen.

Gele werden auch als Träger für Wirkstoffe eingesetzt. Hierbei wird eine gesteuerte Wirkstoffabgabe an die Wunde angerebt. Wirkstoffe bzw. Wirkstoffgruppen, die in diesem Zusammenhang genannt werden, sind beispielsweise: Deoxyribonucleoside, Wachstumsfaktoren (PDGF, TGF, FGF, CGF, CTAP), Protein Kinase C, Antibiotika, Antimykotika, Enzyminhibitoren, Antihistaminika, Antiseptika, Vitamine, Glucocorticoide, antivirale Wirkstoffe, Steroide, Insulin und Analgetika.

DE 38 27 561 C beschreibt den Einsatz von flexiblen, hydrophilen, in Wasser quellbaren Gelfilmen. Zu ihrer Herstellung werden anionaktive Polymere zusammen mit kationenaktiven Polymeren eingesetzt. Zusätzlich sind Feuchthaltemittel, wasserdispergierbare Hilfsstoffe, bis zu 70 % Wasser und Wirkstoff enthalten.

DE 44 46 380 A1 beschreibt Hydrogele, beispielsweise auf der Basis von Gelatine, zur Abgabe von therapeutischen und/oder kosmetischen wirkstoffen an die Haut bzw. zur kosmetischen Pflege und Behandlung empfindlicher Haut- und Nagelstellen. Die beschriebenen Hydrogele sind dadurch gekennzeichnet, daß sie flächenförmig ausgebildete und an Konturen menschlicher Körperstellen angepaßte starrelastische Gebilde sind. Die Hydrogele können durch eine haftklebende Oberfläche gekennzeichnet sein.

WO 98/17252 beschreibt ein in Wasser nicht lösliches, filmbildendes Gel mit (bio)adhäsiven Eigenschaften zur lokalen Abgabe von pharmazeutischen Wirkstoffen. Das Gel enthält mindestens ein wasserunlösliches Cellulosederivat und ein nichtwäßriges Lösungsmittel. Es kann sowohl auf Häuten als auch auf Schleimhäuten appliziert werden.

US 5 770 228, US 4 717 717 und WO 93/08825 beschreiben Gele auf der Basis von Hydroxyethylcellulose. Diese Gele werden zur Abgabe von Wachstumsfaktoren eingesetzt, z.B. PDGF, TGF, EGF und FGF. Zusätzlich können Gele ein Konservierungsmittel, wie beispielsweise Methylparaben oder Ethanol, enthalten.

US 5 705 485 und US 5 457 093 beschreiben Gele zur Behandlung von Wunden. Sie enthalten wirksame Mengen an PDGF (Platelet Derived Growth Factor). Als Gelbildner werden Cellulosederivate, beispielsweise Carboxymethylcellulose, eingesetzt. Ferner enthalten sind Agenzien, die eine dem PDGF entgegengerichtete Ladung tragen. Genannt werden hierbei Aminosäuren wie beispielsweise Lysin oder Alanin, aber auch Metallionen wie Magnesium oder Zink.

US 5 591 709 beschreibt ein Gel als Wirkstoffabgabesystem. Als gelbildende Komponenten werden Gelatine, Agrose, Kollagen und hydrophile Cellulosederivate genannt. Das System dient der Abgabe von Insulin, Wachstumshormonen und Thyroxin bzw. Triiodothyronin.

US 5 578 661 beschreibt ein Gel, das zur Abgabe von PDGF geeignet ist. Es handelt sich dabei um ein wäßriges Gemisch aus mindestens drei Bestandteilen, die zur Gelbilung befähigt sind. Sie enthalten ein hydrophiles, wasserlösliches Polymer als Komponente 1: Polyethylenglycol, Polyvinylpyrrolidon;
ein säurehaltiges Polymer als Komponente 2: Polyvinylpyrrolidon-Polyacryl-säure-Copolymer, Polyacrylsäure, Polymethylvinylether-Polymaleinsäureanhyddrid-Copolymer, Polyethylenmaleinsäureanhydrid;
ein Polymer, welches Aminogruppen aufweist als Komponente 3: z.B. Polysaccharide, Poly-L-Lysin.
Die Säurefunktionen der zweiten Komponente führen zur Quervernetzung und/oder bilden Wasserstoffbrücken zusammen mit der ersten Komponente aus. Dabei dient die dritte Komponente der Aufnahme von Hydroniumionen. Es resultiert ein kohäsives Hydrogel. Kennzeichnend für dieses System ist, daß sich das Gel nicht spontan bildet, sondern erst mit der zeit. Demzufolge ist es möglich, zunächst ein niedrigviskoses Gemisch zu verarbeiten. Das Gemisch härtet jedoch mit der Zeit aus und bildet ein Hydrogel.

US 5 427 778 beschreibt ein Gel zur Abgabe von Wachstumsfaktoren (EGF, FGF, PDGF, TGF, NGF und IGF). Als Gelbildner wird ein Polymer auf der Basis von Acrylamid eingesetzt.

WO 98 28013 A offenbart Wundauflagen bestehend aus einem porösen Behälter, z. B. einem Sachet aus Polyestergewebe oder Baumwoll-Gaze, worin Hydrogelpartikel eingeschlossen sind, die zum Absorbieren von Wundexsudat geeignet sind. Die Hydrogelpartikel können aus verschiedenen Polymeren wie z. B. Polyacrylaten, Polyurethanen oder Polyacrylonitril hergestellt werden und mit Bindern und Weichmachern zu einer Matrix verarbeitet werden. Die absorbierenden Partikel können pharmazeutische Wirkstoffe (z. B. Wachstumsfaktoren, Antibiotika) enthalten. Um eine Befestigung der Wundauflage auf der Haut zu ermöglichen, kann der gesamte Behälter mit einem Klebeband verbunden sein.

US-A-5 705 485 bezieht sich auf wässrige Gelformulierungen für Wunden, wobei diese Formulierungen Wachstumsfaktoren (z. B. Becaplermin) enthalten können und auf eine Bandage aufgebracht werden können. Die Gelformulierungen sind adhäsiv, absorbieren Wundexsudat und ermöglichen die kontrollierte Abgabe des Wachstumsfaktors. Sie enthalten wasserlösliche Polymere, die visköse wässrige Lösungen bilden können, oder wasserunlösliche Polymere, die in Wasser quellfähig sind. Beispielsweise können folgende Polymere Verwendung finden: Vinylpolymere, insbesondere Polyacrylsäure, Polyoxyethylen-Polyoxypropylen-Copolymere, Acrylamid-Polymere, Polysaccharide, insbesondere CelluloseDerivate, Proteine. US-A-5 705 485 beschreibt ferner ein Verfahren zur Herstellung einer Gelformulierung, wobei in einem ersten Schritt ein Hydrogel auf Basis von NatriumCarboxymethylcellulose hergestellt und durch Hitze sterilisiert wird. Anschließend wird ein Wachstumsfaktor unter aseptischen Bedingungen hinzugefügt.

Ein wesentlicher Nachteil der beschriebenen Systeme ist, daß sie durch eine erheblich begrenzte Quellfähigkeit gekennzeichnet sind, und daß sie bei Quellung mehr oder weniger Form und Zusammenhalt verlieren. Deshalb ist nach ihrer Applikation auf Wunden eine intensive Wundreinigung erforderlich. Die Reinigung kann zu einer Reizung oder Störung des Heilungsprozesses führen. Speziell beim Einsatz von hydrokolloiden und Hydropolymeren können sich trotz sorgsamer Reinigung Gelpartikel in der Wunde abkapseln, die möglicherweise zu einer Fremdkörperreaktion führen. Hieraus können sich erhebliche Komplikationen ergeben.

Ausgehend vom vorstehend diskutierten Stand der Technik liegt der Erfindung die Aufgabe zugrunde, eine Wundauflage der im Oberbegriff von Anspruch 1 genannten Art zur steuerbaren Abgabe von Wirkstoff und insbesondere von wundheilungsfördernden Stoffen anzugeben, die unter Volumenzunahme Flüssigkeit aufzunehmen vermag, ohne hierbei ihren Zusammenhalt zu verlieren, und die darüber hinaus den Heilungsprozeß maßgeblich fördert.

Zur Lösung der Aufgabe wird bei der Wundauflage zur steuerbaren Abgabe von Wirkstoffen an Wunden, insbesondere von wundheilungsfördernden Substanzen, mit der Erfindung vorgeschlagen, daß sie zum Zwecke der Aufnahme von Flüssigkeit unter Volumenzunahme, insbesondere zur Aufnahme von Wundexsudat, schichtweise aufgebaut ist und zwei polymerhaltige Schichten, zwei gewebe- oder vliesartige Schichten und mindestens einen Wirkstoff enthält, wobei die Schichten in abwechselnder Reihenfolge übereinander angeordnet und miteinander verbunden sind, und wobei die polymerhaltigen Schichten als Absorber für Flüssigkeit hydrokolloidhaltiges quellfähiges Hydrogel enthalten.

Mit Vorteil wird durch die erfindungsgemäße Ausbildung der Wundauflage erreicht, daß sie einerseits Flüssigkeit wie Wundexsudat in größeren Mengen unter Volumenzunahme aufnehmen kann, ohne ihren Zusammenhalt zu verlieren, und daß durch die andererseits wundheilungsfördernde Stoffe an die Wunde abgegeben werden, wodurch insbesondere unter Berücksichtigung der heilungs fördernden Okklusion der Wunde ein beschleunigter Heilungsprozeß eingeleitet wird, der infolge der Flüssigkeitsaufnahme ein häufiges Wechseln des Verbandes überflüssig macht.

Die erfindungsgemäße wirkstoffhaltige Wundauflage enthält vorteilhaft Hydrokolloidhaltige Hydrogele, die durch die Quellfähigkeit Flüssigkeit aufnehmen können, unter Wahrung ihres Zusammenhaltes. Der Kolloidanteil der Wundauflage bindet dabei nach und nach Exsudat unter Verwandlung in ein freies Gel, welches die Wunde mit ihren Vertiefungen auskleidet.
Im Gegensatz hierzu bildet das Hydrogel eine kohärente Matrix, die besonders in trockenen Wunden aufquellend wirkt und Beläge sowie Nekrosen löst.

Die erfindungsgemäße, wirkstoffhaltige wundauflage dient darüber hinaus der Abgabe von Wachstumsfaktoren an schlecht heilende, chronische Wunden. Beispielsweise sind folgende Wachstumsfaktoren zu nennen: PDGF (Platelet Derived Growth Factor), rhPDGF-BB (Becaplermin), EGF (Epidermal Growth Factor), PDECGF ((Platelet Derived Endothelial Cell Growth Factor), aFGF (Acidic Fibroplast Growth Factor), bFGF (Basic Fibroplast Growth Factor),TGF α (Transforming Growth Factor alpha), TGF β (Transforming Growth Factor beta), KGF (Keratinocyte Growth Factor), IGF1/IGF2 (Insulin-Like Growth Factor) und TNF (Tumor Necrosis Factor).

Wesentliche Ausgestaltungen der Wundauflage nach der Erfindung sind entsprechend den Merkmalen der Unteransprüche vorgesehen, in welchen auch die in den einzelnen Schichten enthaltenen Wirk- und Hilfsstoffe angegeben sind.

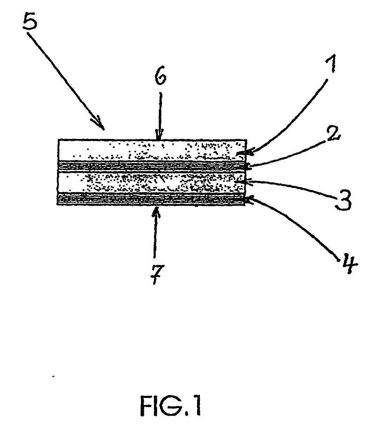

Die Wundauflage nach der Erfindung wird nachfolgend anhand eines in der Zeichnung FIG.1 gezeigten Ausführungsbeispiels näher erläutert und ihre Herstellung beispielhaft angegeben.
Figur 1 zeigt im Schnitt quer zur Ober- bzw. Unterseite die einzelnen Schichten der Wundauflage.

Sie enthält vier Schichten (1-4), und zwar zwei polymerhaltige Schichten (1) bzw. (3) und zwei vlies- bzw. gewebeartige Schichten (2) bzw. (4). Diese bilden insgesamt eine Matrix (5) mit einer Oberseite (6) und einer Unterseite (7) aus.

Die so gebildete Matrix (5) kann sowohl mit der Oberseite (6), die adhäsive Eigenschaften haben kann, als auch mit der Unterseite (7), welche in der Regel nicht klebend ist, appliziert werden.

Für die Herstellung der polymerhaltigen Schicht können alle bekannten biokompatiblen Polymere verwendet werden. Hier sind beispielsweise zu nennen Polyethylenglykolderivate, Polyvinylpyrrolidonderivate, Polymere der Acryl- bzw. Methacrylsäure, Silicone, Polyisobutylene, Polyisoprene, Polystyrole und ähnliche Substanzen.

Die Matrix kann ein Vielfaches ihres Eigengewichtes an Flüssigkeit (wie Wasser oder Exsudat) aufnehmen und dementsprechend anschwellen. Die Aufnahmekapazität wird hierbei durch den eingearbeiteten Absorber sichergestellt. Als Absorber geeignet sind beispielsweise Polymere, wie Cellulose- oder Stärkederivate (beispielsweise Carboxymethylcellulose, Hydroxyethylcellulose, Hydroxypropylcellulose, Bydroxypropylmethylcellulose, Carboxymethylstärke und andere) sowie Polyacrylsäurederivate (z.B. Aquakeep) aber auch niedermolekulare Verbindungen, wie Glycerol, Sorbit oder PEG 400.

Der Zusammenhalt der Auflage wird durch das eingelegte Vlies bzw. Gewebe sichergestellt. Als Vlies bzw. Gewebe sind beispielsweise Materialien auf der Basis von Polyurethan, Polyester, Cellulose oder andere geeignet.

Die Erfindung wird im folgenden anhand eines Beispiels zur Herstellung einer Wundauflage beispielhaft erläutert, die einen Wachstumsfaktor, namentlich PDGF, enthält.

Die wirkstoffhaltige Wundauflage wird in einem zweistufigen Prozeß hergestellt. Dazu wird zunächst die eigentliche Wundauflage mit Techniken, die von der Pflasterfertigung bekannt sind, hergestellt. Im zweiten Schritt wird dann der Wirkstoff in Form einer Lösung zu der Auflage addiert.
Zur Herstellung der eigentlichen Wundauflage wird Carboxymethylcellulose in einer organischen Lösung, bestehend aus Polyvinylpyrrolidon, PEG 400 und Ethanol, dispergiert. Dieses Gemisch wird mit Hilfe eines Auftragwerkes in einer definierten Schichtdicke auf eine silikonisierte Polyesterfolie aufgetragen und 30 min bei 60 °C getrocknet. Die getrocknete Schicht entspricht der polymerhaltigen Schicht (1), wie in FIG.1 dargestellt. Nach dem Trocknungsprozeß wird ein Polyestergewebe (2) auf die polymerhaltige Schicht (1) laminiert. Als Resultat wird ein "Bilayer" erhalten. Zur Präparation des in FIG.1 dargestellten "Multilayer" wird die silikonisierte Polyesterfolie entfernt und die Oberseite der polymerhaltigen Schicht (1) eines "Bilayers" auf die unterseite des Polymergewebes (3) eines zweiten "Bilayers" laminiert. Als Ergbnis wird eine Wundauflage erhalten, die aus vier Schichten (1-4) besteht. Wie bereits erwähnt, wird nach Fertigstellung der eigentlichen Wundauflage (5) die PDGF-haltige Lösung entweder auf die polymerhaltige Schicht (1) oder auf das Polyestergewebe (3) addiert.

Optional kann auch eine sterile PDGF-haltige Wundauflage hergestellt werden. Da PDGF empfindlich gegenüber den herkömmlichen Sterilitätsmethoden ist, muß die Fertigung unter aseptischen Bedingungen erfolgen. Dazu wird, wie oben beschrieben, zunächst die eigentliche Wundauflage (5) gefertigt und einem herkömmlichen temperaturneutralen Sterilisationsverfahren, bevorzugt der Gamma-Sterilisation, unterworfen. Darauf wird die Wirkstofflösung unter aseptischen Bedingungen, beispielsweise unter Einsatz einer sogenannten Laminar Flow Box, zur Wundauflage (5) addiert und abschließend unter aseptischen Bedingungen verpackt.

Die in der Figur 1 im Schnitt dargestellte Wundauflage nach der Erfindung weist vier schichten (1-4) auf, und zwar die polmerhaltigen Schichten (1) und (3), sowie die vlies- bzw. gewebeartigen Schichten (2) und (4). Die polymerhaltige Schicht (1) bildet die Oberseite (6) und die gewebe- oder vliesartige Schicht (4) die Unterseite (7) aus. Jeweils eine dieser Ober- bzw. Unterseiten (6) bzw. (7) kann haftklebend sein.

Im folgenden wird detailliert auf die aseptische Herstellungsweise eingegangen, welche dann notwendig wird, wenn Wirkstoffe verarbeitet werden, die gegenüber herkömmlichen Sterilisationsmethoden empfindlich sind.

Bei dem Verfahren gemäß vorliegender Erfindung wird ein Wirkstoffträger hergestellt, der Wirkstoffträger einem Sterilisationsverfahren unterworfen und mit mindestens einem Wirkstoff unter aseptischen Bedingungen bestückt.

Die Herstellung des Wirkstoffträgers erfolgt nach bekannten Techniken. Als Wirkstoffträger kommen alle Typen von Wundauflagen in Frage. Beispielsweise sind hier zu nennen:
- inaktive Wundauflagen (Mullkompressen, Tupfer, Viskose-Gaze-Kompressen und Saugvlies-Kompressen), die auf zellulärer und stofflicher Ebene nichts verändern, das heißt, sie saugen lediglich mehr oder weniger Sekret auf,
- interaktive Wundauflagen (Hydrokoloide, Hydrokoloid-Kompressen, Hydrogele, Hydrogel-Kompressen, Alginate, Polyurethan-Schaum und -folien), die das Mikroklima in der Wunde verändern, ein physiologisches Milieu schaffen, den Heilungsprozeß beschleunigen, das Sekret absaugen und es speichern,
- aktive Wundauflagen (Aktivkohle-Kompressen, Silber-Aktivkohle-Kompressen und Iodgaze), die durch zytopathische/adsorptive Vorgänge ins Wundklima eingreifen und einen chemisch/physikalisch definierten/gezielten Wirkungsmechanismus haben; sie besitzen in der Regel keine nennenswerte Saugleistung,
- Gaspermeable Systeme (Alginate, Hydrokoloid-Gele, Hyrogele und Spezialfolien), die eine wesentlich höhere Atmungsfähigkeit als die Haut haben. Sie haben ein breites Indikationsgebiet und sind auch bei infizierten Wunden einsetzbar.

Nach Herstellung des Wirkstoffträgers wird dieser einer gängigen Sterilisationsmethode, deren Auswahl sich in erster Linie nach dem zu sterilisierenden Produkt richtet, unterworfen. Da im allgemeinen die pro Zeit-Einheit absterbende Zahl von Mikroorganismen unter konstanten Bedingungen in einem Sterilisationsprozeß von der ursprünglich vorhandenen Anzahl lebensfähiger Keime abhängt, kommt der jeweiligen Ausgangskeimzahl eine große Bedeutung zu. Der Sterilisationsprozeß wird jedoch nicht nur von der Ausgangskeimzahl, sondern in noch höherem Maße von der Art der vorhandenen Mikroorganismen sowie deren Funktionszustand beeinflußt. Die ausgewählte Methode muß einen ausreichenden Wirkungsgrad besitzen. Standardverfahren sind
- Sterilisation mit gespanntem, gesättigtem Wasserdampf (15 min, 2 bar, 121 °C),
- Sterilisation durch trockene Hitze (30 min, 180 °C),
- Strahlensterilisation,
- Gassterilisation.

Es können auch geeignete Äquivalenzverfahren oder Alternativverfahren angewendet werden.

Zu dem sterilisierten Wirkstoffträger wird der Wirkstoff unter aseptischen Bedingungen zugegeben. Die Zugabe kann beispielsweise in Form einer Lösung erfolgen, wobei der Wirkstoff gelöst in sterilisiertem Wasser, einem autosterilen Lösungsmittelgemisch oder einem anderen geeigneten Träger vorliegt.

Unter aseptischem Arbeiten wird das strikte Vermeiden des Einbringens von Keimen während der Herstellung verstanden. Da bei der aseptischen Bereitung die Schlußsterilisation im endgültigen, bakteriendicht verschlossenen Behältnis fehlt, zählt aseptisches Arbeiten nicht zu den Sterilisationsmaßnahmen. Außer einer peinlich sorgfältigen Produngktionshygiene exakter Desinfektion von Räumen und Raumluft gelten heute sogenannte "reine Räume" als Voraussetzung für aseptisches Arbeiten. Unter "reinen Räumen" versteht man Räume oder bestimmte Bereiche in Räumen, in denen durch Filtration der Luft der Grad der Verunreinigung auf höchstens 3,5 Teilchen ≥ 0,5 µm pro Liter Luft herabgesetzt wurde. Bedenkt man, daß bei 1000 Staubteilchen mit einem Keim zu rechnen ist, so kommt in solchen Räumen in etwa 3 m³ ein Keim vor. Bei der Luftfiltration erfolgt die Luftführung so, daß bei einer Geschwindigkeit von etwa 0,5 m/sec ein möglichst laminarer Luftstrom alle Schwebeteilchen aus dem bestrichenen Raum verdrängt. Es können auf diese Weise "reine Bereiche" in sonst normal sauberen Räumen geschaffen werden. Die aseptischen Arbeiten erfolgen dann innerhalb der reinen Bereiche, während sich das Bedienungspersonal außerhalb bewegt und nur bei falschem Verhalten eine Kontaminationsgefahr darstellt.

Das erfindungsgemäße Verfahren wird im folgenden anhand der aseptischen Herstellung wirkstoffhaltiger Hydrogele beschrieben.

Hydrogele sind Feuchtverbände, die Wasser durch Quellen aufnehmen und durch Entquellen abgeben. Sie bestehen aus höhermolekularen Molekülen, die eine kohärente Matrix bilden, welche kleinere Moleküle und wäßrige Lösungen einschließt. Diese Gele sind ausgesprochen konsistenzstabil.

Das Herstellungsverfahren umfaßt die folgenden Schritte:
- Herstellung eines Wirkstoffträgers auf Hydrogelbasis; im folgenden werden zwei Ausführungsbeispiele beschrieben:
   a) Herstellung eines Uni-Laminates: Polyvinylpyrrolidon (2,7 Gew.%) wird in gereinigtem Wasser (34,4 Gew.%) aufgelöst und Glycerin (26,7 Gew.%) sowie Gelatine (15,5 Gew.%) dazugegeben. Die Lösung wird für 10 min quellen gelassen und anschließend bis zur Klärung auf 65 °C erhitzt. Zu dieser Lösung werden nacheinander Saccharose (10,7 Gew.%) und Dexpanthenol (10 Gew.%) unter kontinuierlichem Rühren zugesetzt. Die homogenisierte Lösung wird nachfolgend auf eine geeignete Folie aufgetragen oder in geeignete Behältnisse (z.B. Tiefziehfolien) gefüllt und erstarren gelassen.
   b) Herstellung eines Multi-Laminates: Carboxymethylcellulose wird in einer organischen Lösung, bestehend aus Polyvinylpyrrolidon, PEG 400 und Ethanol, dispergiert. Dieses Gemisch wird mit Hilfe eines Auftragwerkes in einer definierten Schichtdicke auf eine silikonisierte Polyesterfolie aufgetragen und für 30 min bei 60 °C getrocknet. Die getrocknete Schicht wird im folgenden als polymerhaltige Schicht bezeichnet. Nach dem Trocknungsprozeß wird ein Polyestergewebe auf die Oberflächen der polymerhaltigen Schicht laminiert und es resultiert ein "Bi-layer". Zur Präparation eines Multi-Laminates wird die silikonisierte Polyesterfolie entfernt und die Oberseite der polymerhaltigen Schicht eines "Bi-layers" auf die Unterseite des Polymergewebes eines zweiten "Bi-layers" laminiert. Als Ergebnis wird eine Wundauflage erhalten, die aus vier Schichten besteht.
- Abtrennung von Teilmengen
   wird das Hydrogel flächenförmig auf eine Folie aufgetragen, dann werden anschließend mit Hilfe einer geeigneten Stanze einzelne Hydrogelstanzlinge ausgestanzt und diese in geeignete Behältnisse (z.B. Tiefziehfolien) überführt,
- Sterilisationsverfahren
   Die Sterilisation des erstarrten Hydrogels wird mit Hilfe von ionisierenden Strahlen (Strahlensterilisation) durchgeführt. Bevorzugt finden Gamma-Strahlen Anwendung, da diese eine hohe Eindringtiefe haben und nur geringfügig in Wechselwirkung mit dem zu sterilisierenden Material treten. Die anzuwendende Dosis (z.B. 25 kGy) richtet sich hierbei nach der mikrobiologischen Ausgangslage (F=n x D). Als Strahlenquelle dient üblicherweise das Radionuklid ⁶⁰Co.
- Bestückung des Wirkstoffträgers mit Wirkstoff
   Der in sterilisiertem Wasser gelöste Wirkstoff (z.B. PPDGF) wird zu dem sterilisierten Hydrogel dosiert, so daß die Wirkstofflösung die Oberfläche des Wirkstoffträgers gleichmäßig benetzt und von diesem aufgenommen wird.
- Endverpackung
   Die mit Wirkstoffträger und -lösung bestückten Hydrogele werden abschließend verpackt, so daß ein ausreichender Schutz vor mikrobiologischer Rekontamination gewährleistet ist.

## Patentansprüche

1. Wundauflage zur gesteuerten Abgabe von Wirkstoff an Wunden, insbesondere von wundheilungsfördernden Substanzen an schlecht heilende chronische Wunden, **dadurch gekenn**-**zeichnet, dass** sie zum Zwecke der Aufnahme von Flüssigkeit unter Volumenzunahme, insbesondere zur Aufnahme von Wundexsudat, schichtweise aufgebaut ist und zwei polymerhaltige Schichten (1, 3), zwei gewebe- oder vliesartige Schichten (2, 4) und mindestens einen Wirkstoff enthält, wobei die Schichten (1, 2, 3, 4) in abwechselnder Reihenfolge übereinander angeordnet und miteinander verbunden sind, und wobei die polymerhaltigen Schichten (1, 3) als Absorber für Flüssigkeit hydrokolloidhaltiges quellfähiges Hydrogel enthalten.

2. Wundauflage nach Anspruch 1, **dadurch gekennzeichnet, dass** die polymerhaltige Schicht (1, 3) wenigstens ein Polyethylenglykolderivat enthält.

3. Wundauflage nach Anspruch 1, **dadurch gekennzeichnet, dass** die polymerhaltige Schicht (1, 3) wenigstens ein Polymer auf der Basis von Vinylpyrrolidon enthält.

4. Wundauflage nach Anspruch 1, **dadurch gekennzeichnet, dass** die polymerhaltige Schicht (1, 3) wenigstens ein Polymer auf Basis von Acryl- und/oder Methacrylsäure enthält.

5. Wundauflage nach Anspruch 1, **dadurch gekennzeichnet, dass** die polymerhaltige Schicht (1, 3) wenigstens ein Polymer auf Basis von Silicon enthält.

6. Wundauflage nach Anspruch 1, **dadurch gekennzeichnet, dass** die polymerhaltige Schicht (1, 3) wenigstens ein Polymer auf Basis von Isobutylen, Isopren oder Styrol enthält.

7. Wundauflage nach Anspruch 1, **dadurch gekennzeichnet, dass** der Absorber ein Polymer ist.

8. Wundauflage nach Anspruch 7, **dadurch gekennzeichnet, dass** das Polymer ein Cellulosederivat wie Carboxymethylcellulose, ein Stärkederivat oder ein Polyethylenderivat ist.

9. Wundauflage nach Anspruch 1, **dadurch gekennzeichnet, dass** der wundheilungsfördernde Wirkstoff ein biologisch aktives Peptid oder Protein ist.

10. Wundauflage nach Anspruch 1, **dadurch gekennzeichnet, dass** das biologisch aktive Peptid oder Protein ein Wachstumsfaktor ist.

11. Wundauflage nach Anspruch 10, **dadurch gekennzeichnet, dass** der Wachstumsfaktor Platelet Derived Growth Factor (insbesondere Becaplermin), Epidermal Growth Factor, Platelet Derived Endothelial Cell Growth Factor, Acidic Fibroblast Growth Factor, Basic Fibroblast Growth Factor, Transforming Growth Factor alpha, Transforming Growth Factor beta, Keratinocyte Growth Factor, Insulin-Like Growth Factor 1 bzw. 2 oder Tumor Necrosis Factor ist.

12. Wundauflage nach Anspruch 1, **dadurch gekennzeichnet, dass** sie ein Gewebe oder Vlies auf Basis von Polyester, Polyurethan oder Cellulose enthält.

13. Wundauflage nach einem der vorhergehenden Ansprüche, **dadurch gekennzeichnet, dass** ihre zur Applikation vorgesehene Oberfläche adhäsive Eigenschaften aufweist.

14. Wundauflage nach Anspruch 1, **dadurch gekennzeichnet, dass** sie
(1) mindestens eine polymerhaltige Schicht (1, 3), enthaltend 40-80 Gew.% Polyvinylpyrrolidon, 15-45 Gew.% Polyethylenglycol 400 und bis zu 40 Gew.% NatriumCarboxymethylcellulose,
(2) mindestens eine vliesartige Schicht (2, 4) enthaltend Langfaserpapier mit einem Flächengewicht von 5-100 g/m² und
(3) Becaplermin als Wirkstoff
enthält.

15. Wundauflage nach Anspruch 14, **dadurch gekennzeichnet, dass** sie
(1) mindestens eine polymerhaltige Schicht (1, 3), enthaltend 60 Gew.% Polyvinylpyrrolidon, 35 Gew.% Polyethylenglycol 400 und 5 Gew.% Natrium-Carboxymethylcellulose,
(2) mindestens eine vliesartige Schicht (2, 4) enthaltend Langfaserpapier mit einem Flächengewicht von 17 g/m² und
(3) Becaplermin als Wirkstoff
enthält.

16. Verfahren zur Herstellung einer Wundauflage nach einem der Ansprüche 1 bis 15, **dadurch gekennzeichnet, dass**
- durch Auftragen einer ein Hydrogel enthaltenden polymerhaltigen Schicht (1) auf eine gewebe- oder vliesartige Schicht (2) und nachfolgendes Trocknen ein zweischichtiges Laminat (1, 2) hergestellt wird;
- zwecks Herstellung eines Wirkstoffträgers das zweischichtige Laminat mit einem weiteren, auf dieselbe Weise hergestellten zweischichtigen Laminat (3, 4) verbunden wird, so daß ein vierschichtiges Laminat gebildet wird, in welchem die Schichten (1, 2, 3, 4) in abwechselnder Reihenfolge übereinander angeordnet sind;
- der so erhaltene Wirkstoffträger einem Sterilisationsverfahren unterworfen wird und
- der Wirkstoffträger unter aseptischen Bedingungen mit mindestens einem Wirkstoff versehen wird.

17. Verfahren nach Anspruch 16, **dadurch gekennzeichnet, dass** ein einen Wirkstoff enthaltender Wirkstoffträger hergestellt wird.

18. Verfahren nach Anspruch 16, **dadurch gekennzeichnet, dass** das Sterilisationsverfahren nach der Methode der Hitzesterilisation, der Strahlensterilisation oder der Gassterilisation durchgeführt wird.

19. Verfahren nach Anspruch 16, **dadurch gekennzeichnet, dass** der Wirkstoff in Form einer Lösung, insbesondere einer wässrigen Lösung, zu dem Wirkstoffträger dosiert wird.

20. Verfahren nach Anspruch 18, **dadurch gekennzeichnet, dass** der Wirkstoff bei Anwendung der Sterilisationsmethoden instabil ist.

21. Verfahren nach Anspruch 16, **dadurch gekennzeichnet, dass** der Wirkstoff ein Wachstumsfaktor ist.

22. Verfahren nach Anspruch 16, **dadurch gekennzeichnet, dass** der Wirkstoff Platelet Derived Growth Factor (PDGF) ist.

23. Verfahren nach Anspruch 16, **dadurch gekennzeichnet, dass** der wirkstoffhaltige Träger verpackt wird.

## Claims

1. Wound dressing for the controlled release of active substance to wounds, especially of wound healing-promoting substances to slow-healing, chronic wounds, **characterized in that** said wound dressing has a layered structure for the purpose of absorbing liquid, especially wound exudate, under volume increase, and that said wound dressing comprises two polymer-containing layers (1, 3), two woven fabric-like or nonwoven-like layers (2, 4) and at least one active substance, said layers (1, 2, 3, 4) being arranged in alternating sequence one upon the other and being connected with one another, and said polymer-containing layers (1, 3) containing hydrocolloid-containing swellable hydrogel as absorbent for liquid.

2. Wound dressing according to Claim 1, **characterized in that** the polymer-containing layer (1, 3) contains at least one polyethylene glycol derivative.

3. Wound dressing according to Claim 1, **characterized in that** the polymer-containing layer (1, 3) contains at least one polymer based on vinyl pyrrolidone.

4. Wound dressing according to Claim 1, **characterized in that** the polymer-containing layer (1, 3) contains at least one polymer on the basis of acrylic and/or methacrylic acid.

5. wound dressing according to Claim 1, **characterized in that** the polymer-containing layer (1, 3) contains at least one polymer based on silicone.

6. Wound dressing according to Claim 1, **characterized in that** the polymer-containing layer (1, 3) contains at least one polymer based on isobutylene, isoprene or styrene.

7. Wound dressing according to Claim 1, **characterized in that** the absorber is a polymer.

8. Wound dressing according to Claim 7, **characterized in that** the polymer is a cellulose derivative such as carboxymethyl cellulose, a starch derivative or a polyethylene derivative.

9. Wound dressing according to Claim 1, **characterized in that** the wound healing-promoting active substance is a biologically active peptide or protein.

10. Wound dressing according to Claim 1, **characterized in that** the biologically active peptide or protein is a growth factor.

11. Wound dressing according to Claim 10, **characterized in that** the growth factor is Platelet Derived Growth Factor (especially Becaplermin), Epidermal Growth Factor, Platelet Derived Endothelial Cell Growth Factor, Acidic Fibroblast Growth Factor, Basic Fibroblast Growth Factor, Transforming Growth Factor alpha, Transforming Growth Factor beta, Keratinocyte Growth Factor, Insulin-Like Growth Factor 1 or 2, or Tumor Necrosis Factor.

12. Wound dressing according to Claim 1, **characterized in that** it comprises a woven fabric or nonwoven based on polyester, polyurethane or cellulose.

13. Wound dressing according to any one of the preceding claims, **characterized in that** the surface thereof which is intended for application possesses adhesive properties.

14. Wound dressing according to Claim 1, **characterized in that** it contains:
(1) at least one polymer-containing layer (1, 3) containing 40 - 80%-wt. of polyvinyl pyrrolidone, 15 - 45%-wt. of polyethylene glycol 400, and up to 40%-wt. of sodium carboxymethyl cellulose,
(2) at least one nonwoven-like layer (2, 4) containing long-fibre paper having a weight per unit area of 5 - 100 g/m², and
(3) becaplermin as active substance.

15. Wound dressing according to Claim 14, **characterized in that** it contains:
(1) at least one polymer-containing layer (1, 3) containing 60%-wt. of polyvinyl pyrrolidone, 35%-wt. of polyethylene glycol 400, and 5%-wt. of sodium carboxymethyl cellulose,
(2) at least one nonwoven-like layer (2, 4) containing long-fibre paper having a weight per unit area of 17 g/m², and
(3) becaplermin as active substance.

16. Process for manufacturing a wound dressing according to any one of claims 1 to 15, **characterized in that**
- a bilayer laminate (1, 2) is produced by applying a polymer-containing layer (1), containing a hydrogel, to a woven fabric-like or nonwoven-like layer (2) and subsequent drying;
- to produce an active substance carrier, the two-layered laminate is connected with a further bilayer laminate (3, 4), which has been prepared in the same manner, so that a four-layer laminate is formed, wherein the layers (1, 2, 3, 4) are arranged in alternating sequence one upon the other;
- the active substance carrier thus obtained is subjected to a sterilisation process, and
- the active substance carrier is equipped under aseptic conditions with at least one active substance.

17. Process according to Claim 16, **characterized in that** an active substance carrier containing an active substance is prepared.

18. Process according to Claim 16, **characterized in that** the sterilisation process is performed according to the heat sterilisation, radiation sterilisation or gas sterilisation methods.

19. Process according to Claim 16, **characterized in that** the active substance is dosed to the active substance carrier in the form of a solution, especially an aqueous solution.

20. Process according to Claim 18, **characterized in that** the active substance is unstable when the said sterilisation methods are employed.

21. Process according to Claim 16, **characterized in that** the active substance is a growth factor.

22. Process according to Claim 16, **characterized in that** the active substance is a Platelet Derived Growth Factor (PDGF).

23. Process according to Claim 16, **characterized in that** the active substance-containing carrier is packaged.

## Revendications

1. Pansement pour la libération contrôlée d'une substance active au niveau de plaies, en particulier de substances favorisant la guérison de la plaie au niveau de plaies chroniques qui guérissent mal, **caractérisé en ce qu'**il présente une structure à couches en vue de l'absorption d'un liquide avec une augmentation de volume, en particulier de l'absorption d'un exsudat de la plaie et contient deux couches (1, 3) contenant un ou plusieurs polymères, deux couches (2, 4) de type tissu ou non-tissé et au moins une substance active, les couches (1, 2, 3, 4) étant disposées alternativement les unes au-dessus des autres et reliées les unes aux autres, les couches (1, 3) contenant un ou plusieurs polymères contenant, comme absorbant pour le liquide, un hydrogel pouvant gonfler contenant un hydrocolloïde.

2. Pansement selon la revendication 1, **caractérisé en ce que** la couche (1, 3) contenant un ou plusieurs polymères contient au moins un dérivé de polyéthylèneglycol.

3. Pansement selon la revendication 1, **caractérisé en ce que** la couche (1, 3) contenant un ou plusieurs polymères contient au moins un polymère à base de vinylpyrrolidone.

4. Pansement selon la revendication 1, **caractérisé en ce que** la couche (1, 3) contenant un ou plusieurs polymères contient au moins un polymère à base d'acide acrylique et/ou méthacrylique.

5. Pansement selon la revendication 1, **caractérisé en ce que** la couche (1, 3) contenant un ou plusieurs polymères contient au moins un polymère à base de silicone.

6. Pansement selon la revendication 1, **caractérisé en ce que** la couche (1, 3) contenant un ou plusieurs polymères contient au moins un polymère à base d'isobutylène, d'isoprène ou de styrène.

7. Pansement selon la revendication 1, **caractérisé en ce que** l'absorbant est un polymère.

8. Pansement selon la revendication 7, **caractérisé en ce que** le polymère est un dérivé de cellulose, tel que la carboxyméthylcellulose, un dérivé d'amidon ou un dérivé de polyéthylène.

9. Pansement selon la revendication 1, **caractérisé en ce que** la substance active favorisant la guérison de la plaie est un peptide biologiquement actif ou une protéine biologiquement active.

10. Pansement selon la revendication 1, **caractérisé en ce que** le peptide biologiquement actif ou la protéine biologiquement active est un facteur de croissance.

11. Pansement selon la revendication 10, **caractérisé en ce que** le facteur de croissance est le facteur de croissance dérivé des plaquettes (en particulier le Becaplermin), le facteur de croissance épidermique, le facteur de croissance de cellules endothéliales dérivé des plaquettes, le facteur fibroblastique de croissance acide, le facteur fibroblastique de croissance basique, le facteur de croissance de transformation alpha, le facteur de croissance de transformation bêta, le facteur de croissance de kératinocyte, le facteur de croissance de type insuline 1 ou 2 ou le facteur de nécrose tumorale.

12. Pansement selon la revendication 1, **caractérisé en ce qu'**il contient un tissu ou un non-tissé à base de polyester, de polyuréthane ou de cellulose.

13. Pansement selon l'une quelconque des revendications précédentes, **caractérisé en ce que** la surface prévue pour l'application présente des propriétés adhésives.

14. Pansement selon la revendication 1, **caractérisé en ce qu'**il contient
(1) au moins une couche contenant un ou plusieurs polymères (1, 3), contenant 40-80% en poids de polyvinylpyrrolidone, 15-45% en poids de polyéthylèneglycol 400 et jusqu'à 40% en poids de carboxyméthylcellulose de sodium
(2) au moins une couche de type non-tissé (2, 4) contenant un papier à longues fibres présentant un grammage de 5-100 g/m² et
(3) du Becaplermin comme substance active.

15. Pansement selon la revendication 14, **caractérisé en ce qu'**il contient
(1) au moins une couche (1, 3) contenant un ou plusieurs polymères, contenant 60% en poids de polyvinylpyrrolidone, 35% en poids de polyéthylèneglycol 400 et 5% en poids de carboxyméthylcellulose de sodium,
(2) au moins une couche de type non-tissé (2, 4) contenant un papier à longues fibres présentant un grammage de 17 g/m² et
(3) du Becaplermin comme substance active.

16. Procédé pour la réalisation d'un pansement selon l'une quelconque des revendications 1 à 15, **caractérisé**
- **en ce qu'**on réalise, par application d'une couche (1) contenant un ou plusieurs polymères, contenant un hydrogel, sur une couche (2) du type tissu ou non-tissé et séchage consécutif, un stratifié à deux couches (1, 2) ;
- **en ce qu'**on assemble, en vue de la réalisation d'un support de substance active, le stratifié à deux couches avec un autre stratifié (3, 4) à deux couches réalisé de la même manière, de manière à former un stratifié à quatre couches, dans lequel les couches (1, 2, 3, 4) sont disposées alternativement les unes au-dessus des autres ;
- **en ce que** le support de substance active ainsi obtenu est soumis à un procédé de stérilisation et
- **en ce que** le support de substance active est pourvu d'au moins une substance active dans des conditions aseptiques.

17. Procédé selon la revendication 16, **caractérisé en ce qu'**on prépare un support de substance active contenant une substance active.

18. Procédé selon la revendication 16, **caractérisé en ce que** le procédé de stérilisation est réalisé selon le procédé de la stérilisation par la chaleur, par des rayons ou par un gaz.

19. Procédé selon la revendication 16, **caractérisé en ce que** la substance active est dosée sous forme d'une solution, en particulier d'une solution aqueuse, dans le support de substance active.

20. Procédé selon la revendication 18, **caractérisé en ce que** la substance active est instable lors de l'utilisation des procédés de stérilisation.

21. Procédé selon la revendication 16, **caractérisé en ce que** la substance active est un facteur de croissance.

22. Procédé selon la revendication 16, **caractérisé en ce que** la substance active est le facteur de croissance dérivé des plaquettes (Platelet Derived Growth Factor - PDGF).

23. Procédé selon la revendication 16, **caractérisé en ce que** le support contenant la substance active est emballé.
